# EUROPEAN PATENT SPECIFICATION

(11) **EP 3 463 408 B1**
(45) Date of publication and mention of the grant of the patent: **29.01.2020**
(21) Application number: 17745201.8
(22) Date of filing: 31.05.2017
(51) Int. Cl.: A61K 36/35, A61P 1/04, A61P 17/00, A61P 27/02, A61P 31/22

(54) **EXTRACT FROM BRANCHES OF SAMBUCUS NIGRA L, A PROCESS FOR THEIR PRODUCTION AND THEIR USES**
EXTRAKT AUS ZWEIGEN VON SAMBUCUS L NIGRA, VERFAHREN ZU DESSEN HERSTELLUNG UND DESSEN VERWENDUNGEN
EXTRAIT DE BRANCHES DE SAMBUCUS NIGRA L, UN PROCÉDÉ DE PRODUCTION ET SES UTILISATIONS

(30) Priority: 01.06.2016 IT UA20164041
(43) Date of publication of application: 10.04.2019
(73) Proprietor: Sale, Costantino, 07030 Santa Maria Coghinas SS (IT)
(72) Inventor: MARCHETTI, Mauro, 07100 Sassari (IT); USAI, Marianna, 07043 Bonnanaro SS (IT); SALE, Costantino, 07030 Santa Maria Coghinas SS (IT)
(74) Representative: Germinario, Claudio
(86) International application number: PCT/IB2017/053204
(87) International publication number: WO 2017/208170

(56) References cited:
- EP-A1- 0 699 443
- WO-A1-2012/033422
- WO-A2-2007/113892

## Description

The present invention relates to a process for the preparation of an extract from branches of *Sambucus nigra L,* the so-obtained extract and the use thereof in a series of dermocosmetic preparations. The extracts were characterized by the chemical analysis, by allowing to identify a series of active principles capable of mitigating significantly some cutaneous alterations, due to small wounds, bacterial infections, even serious sun erythema and insect bites, moreover some of these substances are capable of mitigating the cutaneous blemishes caused by exanthematic diseases. The formulations for the preparations of creams, gel, sprays, mouth wash and liquid soap even for the intimate hygiene were identified.

### State of art

There are several biochemical and chemical studies about the active principles of *Sambucus nigra* L; in the bark α-amyrenone (URS-12-en-3-one), α-amyrin, betulin, oleanolic acid, beta-sitosterol [3] were insulated, as well as β-nigrin, a lectin similar to ricin, and 2 other proteins inactivating ribosomes (RIPs) which are less toxic for the cells and the animals .[4]

**The flowers** / **leaves** include flavonoids thereamong quercetin (up to 3%), rutin,iperosid [5], and the anthocyanins [6], as well as essential oils (origin of the typical aroma of muscat of elder flowers) [7], mucilage, tannins (3%), organic acids, glucosides (0.042% in percentage), plastocyanin [8], and sambunigrin (0,042% in percentage). High amounts of amides of L-phenylpropenoyl amino acids were found in the flowers of *Sambucus nigra*.[9]

**The fruit** of *S. nigra* includes the agglutinin protein FIV or SNAIVf, which is homologous of the protein of the type inactivating the ribosome (RIP)[10], whereas the **bark** includes again a protein of type 2 RIP (SNLRP), constituted by a A-chain with activity on the N-glicosidase and a B-chain with action on the bonds usually present on the carbohydrates [11], [12]. Two additional RIPs were detected in the **bark** (SNAI and SNAI ')[11], to demonstrate the complexity of the 2 RIP type of lectines of *S. nigra.* The lectin insulated from the bark is tetrameric with two distinct sub-units and it is enriched with glutamine / glutamic acid, valine, leucine etc. [13]

The fruit of *Sambucus nigra* is a lectin of 2 RIP type with 10 aminoacids, longer than the lectin of bark.[14]. The above-mentioned RIP with action on N-glicosidase is suitable to inhibit the synthesis of the proteins in rubbits, but not in plants.[15].

Quercetin is present in elder in good concentration and it was demonstrated that it is a powerful inhibitor of xanthine oxidase.[16]

EP 0 699 443 B1 ("Elder-containing skin preparation" describes the use of an elder extract, for the preparation of a medicament suitable for the treatment of lesions, in particular burns, sores, sun erythemas, on the human or animal body. Such extract is obtained from the branches treated with hot water 80-100°C at atmospheric pressure generally for a period of time comprised between 15 minutes and 10 hours, preferably for periods of time variable between 1 and 2 hours. Examples of applications on burns and on solar erhythemas are shown.

WO 2007/113892 A2 (*"Use of Sambucus nigra L. decoction* as *antioxidant*") substantially uses the extract of the previous patent, by showing exeriments which demonstrate the antioxidant activity of the extract and claiming the use thereof as drug antioxidant for the treatment of the skin in wounds, as protection from UV rays, as anti-inflammatory treatment, antiedematous analgesic, as activator of hair regrowth and anti-aging. It also claims a dermocosmetic use thereof if it is stabilized with agents and excipients, not specificying further.

The mentioned documents limit to describe procedures which lead to obtain extracts which do not include all active molecules of the starting material, which fact causes both an insufficient and limited biological activity of the same extract and, consequently, a use which limits to soothing of burns and decubitus plaques and problems of stabilizing the same extracts.

Therefore, in the state of art the need was felt to make available an improved and stable elder extract which would allow even the treament of disorders which cannot be treated with the extracts of known art.

Now it has been surprisingly found that an extraction process which provides a *step* of extraction under pressure and at high temperature in autoclave - without exposure to air - succeeds in solving the problems of the state of art.

Therefore the present invention is defined in claims 1-10.

The extract thereto the present invention relates, thanks to its increased effectivness due to the additional active compounds contained therein, such as for example lignans, betulinic acid, salvianolic acid and the ginsenosides, is capable of acting in a more effective way against the disorders treated with the extracts of the state of art and with surprising effectiveness on a whole series of skin and mucous inconveniences which cannot be soothed with the decoctions described in the prior art. Moreover, the different composition and the increased power of the bioactive action of the extract thereto the present invention relates allowed the use thereof even in cosmetic preparations.

### Brief description of the figures

Twenty-two tables are enclosed to the present invention, which represent:
Figure 1 an extract obtained by using the procedure described in WO 2007/113892
Figure 2 an extract obtained in Methanol;
Figure 3 fractions deriving from the distribution of an acidified methanol extract of *S*. *nigra* L. (**S1** = ethyl acetate fraction; **S2** = butanol fraction; **S3** = water fraction);
Figure 4 HPLC chromatogram of the extract which can be obtained from the process of the present invention;
   All figures represent the chromatogram trace of the extracts registered at 280 nm of wavelength.
Figure 5 shows a hand burned with hot oil, treated with the extract according according to the invention 1 day after burn:
Figure 6 the situation of the hand of figure 5 1 day after burn;
Figure 7a e Figure 7b the situation of the hand of figure 5 some days after burn;
Figure 8 the situation of the hand of figure 5 10 days after burn;
Figure 9 the situation of the hand of figure 5 at the beginning of healing 15 days after burn;
Figure 10 the situation of the hand of figure 5 after about 18 days;
Figure 11 the situation of the hand of figure 5 showing healing after 22 days;
Figure 12 the situation of the hand of figure 5 showing healing after 25 days;
Figure 13 shows the scalp with eczema at the beginning of the treatment;
Figure 14 shows the scalp after two days of treatment with a spray according to the present invention;
Figure 15 shows the scalp after one week of treatment;
Figure 16 shows the scalp at the end of treatment;
Figure 17 shows an eye suffering from viral conjunctivitis treated with the decoction according to the present invention for 1 day;
Figure 18 the eye on the 3rd day of application of a decoction according to the invention by means of sterile gauze compresses;
Figure 19 the eye on the 5th day;
Figure 20 the eye on the 7th day;
Figure 21 the eye on the 10th day after complete healing.

The extracts which can be obtained according to the process of the invention were all characterized both qualitatively, and quantitatively. The existing active principles were evaluated in the biological properties thereof and this knowledge allowed to optimize the process according to the invention.

### Description of the invention

The process according to the invention includes the following procedures
a. arrangement of two-year-old branches of *Sambucus nigra L.* (including from 20 to 50% by weight of younger branches in vegetative status) and possible grinding thereof;
b. addition of deionized water and extraction of the material obtained from the procedure a) in autoclave without exposure to air under pressure between 2 and 5 atm, preferably between 3 and 4 atmospheres, for time periods varying from 1 to 5 hours and at a temperature of 120 - 150°C, using a weight ratio of material obtained from stage a): deionized water from 1:10 to 1:20;
c. cooling to room temperature for a period of 5 - 24 hours and collection of the aqueous extract;
d. filtration on a filter having a porosity so as to prevent the passage of the sediments advantageously with 1 micron to 10 micron and obtainment of a filtered extract.

### Comparison between the extraction methods

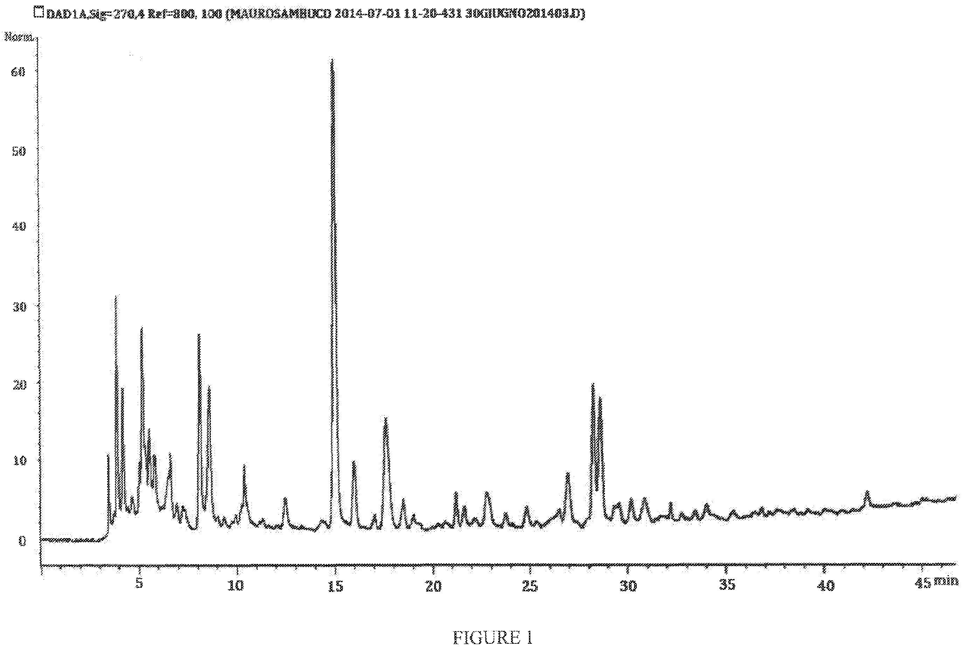

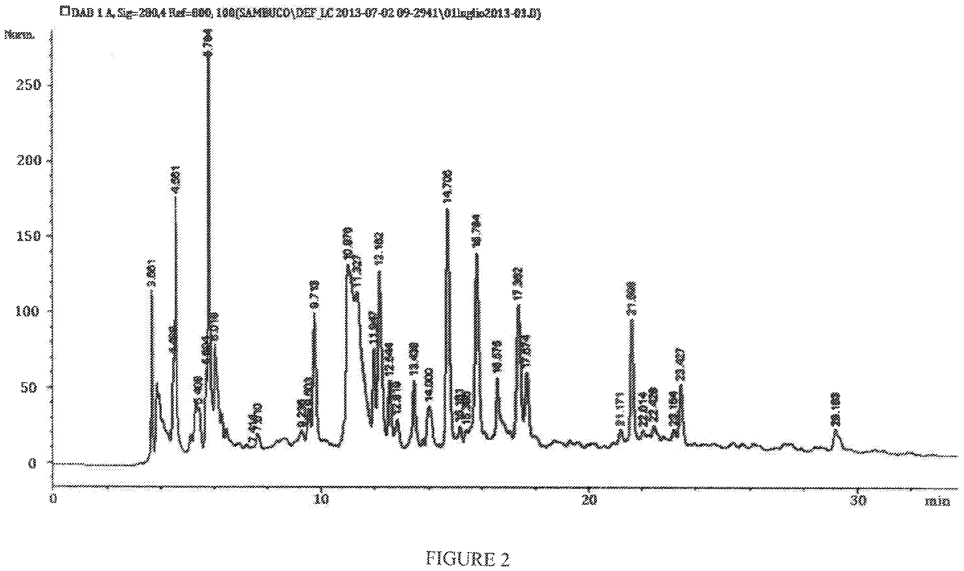

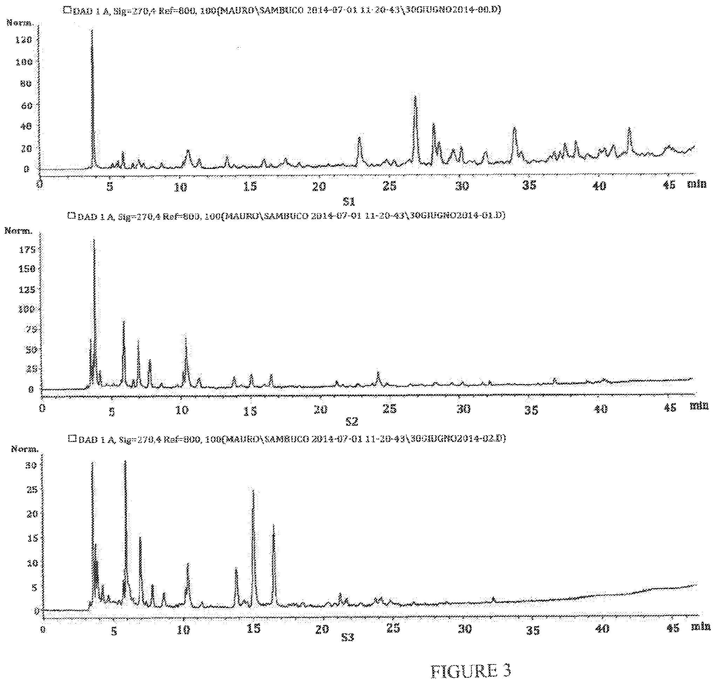

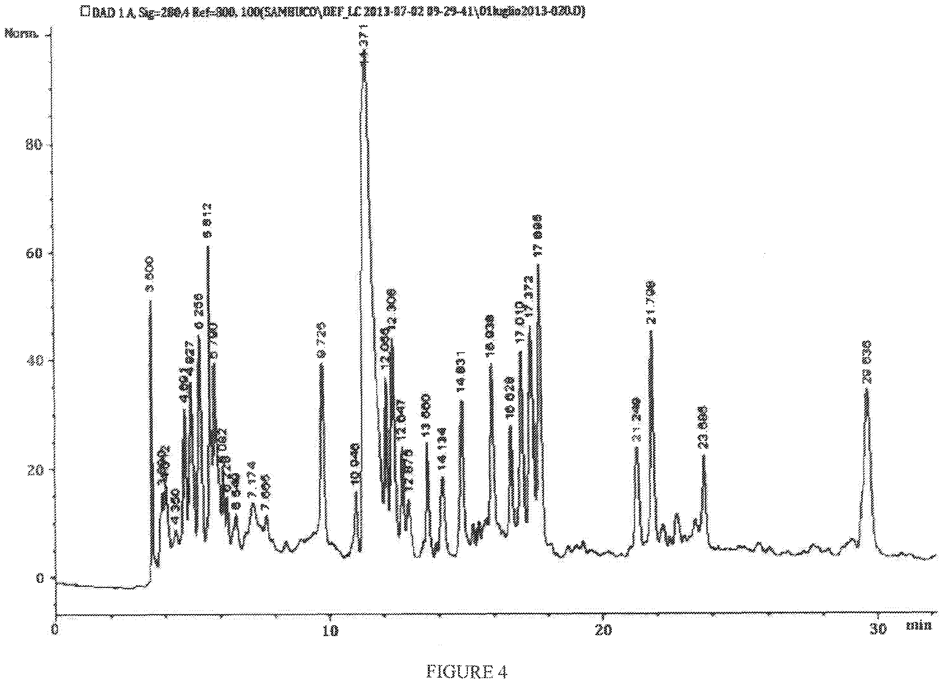

With the purpose of demonstrating the uniqueness of extract which can be obtained according to the process of the invention and the difference from the extracts known from the state of art the following various extractions modes were used:
1. extraction according to WO 2007/113892, HPLC is shown in figure 1
2. extraction in methanol (see figure 2).
3. extraction in acidified Methanol (1% HCl) disributed in three fractions with solvents having different polarity (see figure 3).
4. extraction according to the process of the invention (see figure 4).

From the comparison of the chromatographic profiles of the various extractive methods under the various experimental conditions the following conclusions can be deducted.

### 1. Extract obtained by using the procedure described in WO 2007/113892

As it can be observed from the chromatogram of Figure 1 the extraction, which however involves even practical difficulties due to strong foaming, did not allow to obtain several of the active principles existing in the extract related to the extraction in autoclave under pressure. The class of compounds which is obtained in an effective way through this route limits to that of polyphenols. It is to be noted that apart from the different composition even the extract quantity is much lower than that obtained with the extraction under pressure.

### 2. Extract obtained in Methanol

As it can be observed from the chromatogram of Figure 2 some variations occurred with respect to the composition of the extract under pressure (figure 4). In this case the fraction constituted by the phenolic acids was not subjected to considerable variations compared to the existing one in the extract under pressure, but the polyphenolic fraction, and in particular the glycosyl flavonoids highlighted considerable, both qualitative and quantitative, composition differences.

In particular, the main compound existing in the extract according to the present invention (Apigenin 7-O-beta-D-glucoside), under these experimental conditions is subject to a strong decomposition, in fact the presence of 4',5-dihydroxyflavone (PM 254) was noted, deriving from the decomposition thereof, with loss of one glucose molecule.

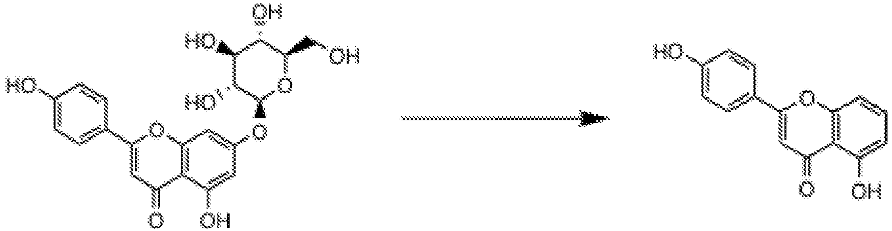

Almost the whole polyphenolic fraction showed a considerable composition variation with the disappearance of some products important for the biological activity of the extract, in particular for the anti-inflammatory and antibiotic activity. Even the decomposition of the salvianolic acid B, so that it is possible to assume an additional loss in the anti-inflammatory and antitumoral power of the preparation.

### 3. Extraction in acidified Methanol (1% HCI) distributed in three fractions with solvents with different polarity

This extractive mode was used to be able to distribute in different fractions the methabolic extract, with the purpose of obtaining fractions with different biological activities, even in this case we noted the same losses of active principles which occurred with the extraction in not acidified methanol, furthermore the presence of not known products, extraneous to the vegetable metabolic pathways, probably due to synthetic artifacts caused by the presence of traces of acid in the extractive means.

### 4. Extraction in autoclave under pressure (2-5 atm; 120-150°C) according to the present invention

Surprisingly the process of the invention, which provides a step of treatment at high pressure in autoclave, provided the best results. To this regard it has to be highlighted that in the extraction of natural products from plants, the person skilled in the art is not induced to think about an increase in temperature. In fact, most part of the products of glycosidic and phenolic origin included in the natural products can be easily subjected to decompositive processes, which invalidate the biological activity thereof. This fact is so known that, in the concentration process (removal of water and/or solvent) of the natural extracts from vegetable products, temperatures higher than 50-60°C are never used and one acts even under light depression. The originality of the invention process mainly lies in the treatment at raised temperature and under pressure. The slow cooling without opening the autoclave and then without an oxygen excess comes in contact to the overheated extract, allows to avoid oxydation and hydrolisys which are extremely favoured at high temperature.

The thermal degradation of the polyphenols already at temperatures near the boiling temperature of water, is a fact known in literature. By way of example the article of I. Volf et. al. of 2014 [17] confirms what scientifically is commonly known, that is the thermal degradation of 30% at 100°C of the polyphenols, which are mainly the active principles of the extract of the present invention. Among the other several publications which faced the same phenomenon a work on dyes extracted from eldberries can be mentioned, as shown in the article published by A. Jose et. al. in 2013 [18], wherein a significant degradation of anthocyanins already at 90°C is reported. The Official Pharmacopei XII_edition, in case of production of aqueous extracts mentions "adequate temperatures: so as to allow the not degradation of the active compounds". What is above described, shown in literature, was confirmed by us with the present invention, in fact the extract reported in the previous invention by *Solinas et. al.,* obtained at the temperature of 80-100°C, has far fewer active principles than our extract obtained with methanol at 60°C, then once again it is necessary to underline that it is surprising the fact that what obtained in the present invention at much higher temperatures is better in quantity and quality of active principles, practically the pressure and the not contact with oxygen allowed to obtain an extract wholly new in composition and activity. In the light of what above illustrated it is clear that an expert in the art, knowing what reported in literature and in publications of general common knowledge (such as the Pharmacopeia), would never have thought to obtain an aqueous extract of Elderberry wood **improved** under the profile of the type and quantity of antioxidant substances, in particular polyphenols (as written in the prior art, but even as it is well defined by our analyses) and **stable** by increasing - and to a so significant extent - the temperature of extraction water.

Since it is a process different from those of the state of art even the obtained extract (see figure 4) has to be considered different than those of the state of art.

### Advantages

The advantages of the present invention can be shown as:
- Total extraction of the active principles with increasing in active molecules with respect to past and consequent possibility of precise molecular characterization and standardization of the extracts, which are equipped with a series of surprising effects demonstrated by the not expected capability of soothing and solving a series of inconveniences of skin and mucosae such as for example acne, sores in the mouth, throat plaques, internal fissures and hemorrhoids, herpes zoester, herpes labiale simplex, blemishes due to chickenpox and rubella, redness of the eyes, perianal or sacro-coccygeal fistulas, rashes in the head among hair, intimate itching and anal itching, gastric problems.
- Possibility of fully exploiting the properties of the active principles of the branches of S. *nigra* through dermocosmetic preparations aimed at the treatment of acne and wrinkles, post shaving rednesses, loss of hair, skin blemishes, skin rednesses of various nature, chapped lips, dermatitis and diaper rash, sun erythema, chapping and sores cutanee;
- Stabilization of the extracts based upon their molecular content.

### Characterization of the components of the extract according to the invention

The extract according to the process of the invention was analyzed by using a HPLC/MS. As it can be noted from the profile shown in figure 4, the composition is quite complex, however more than 90% of the existing products were identified. Such compounds resulted to be very interesting and, as it will be seen later, show the different biological activities attributed to the plant under examination, as shown in ethnobotany. The identified compounds are shown hereinafter.

It is to be noted that some categories of products are not typically present in common vegetable extracts, for example, in the extract according to the invention there is a series of molecules with recognized antiproliferative activity, such as the betulinic acid and strong anti-inflammatory agents such as the lignans, existing in definitely important quantities and in the active forms thereof.

### Saponins

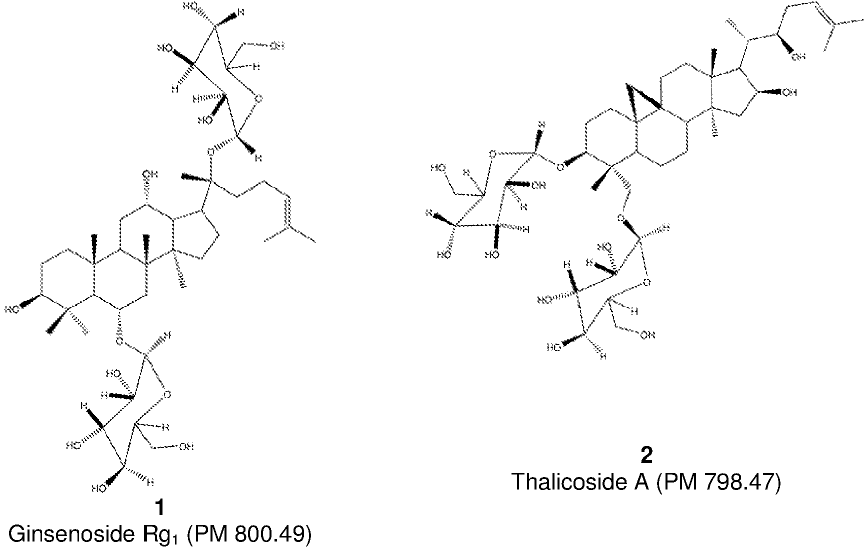

### Acids

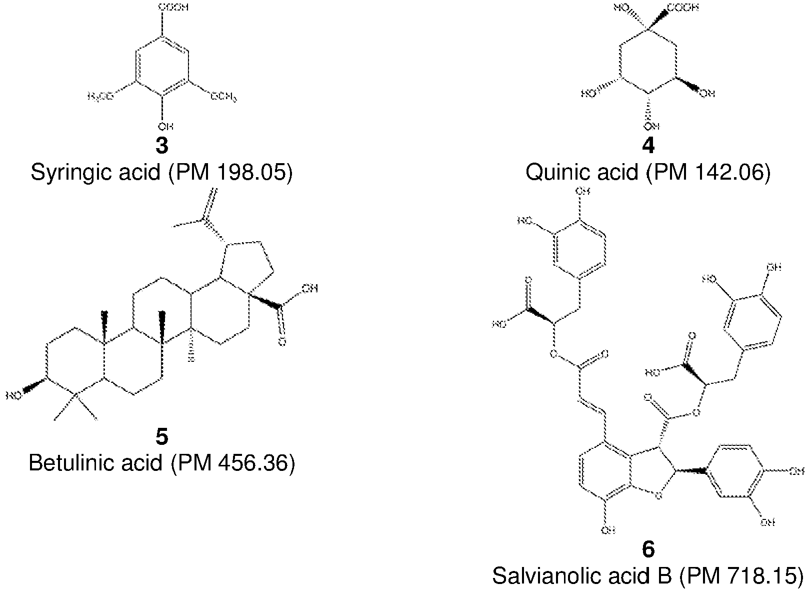

### Lignans

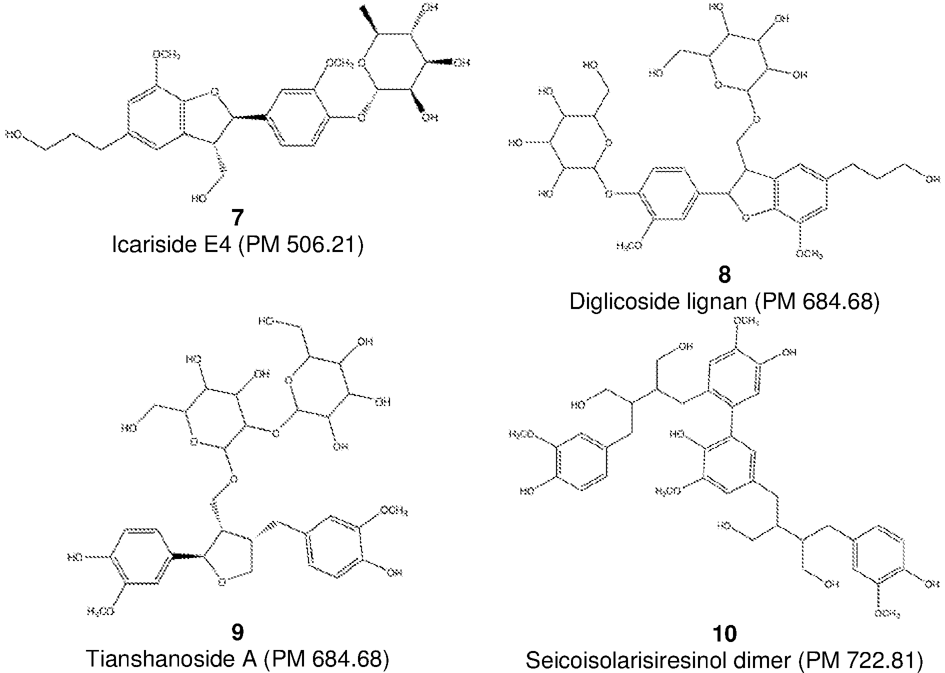

### Polyphenols

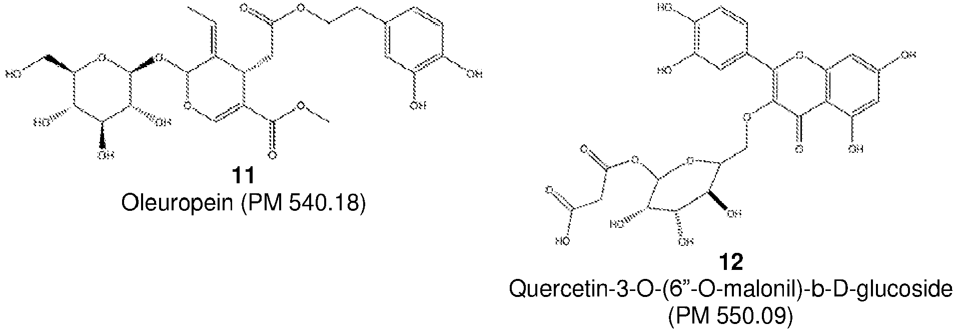

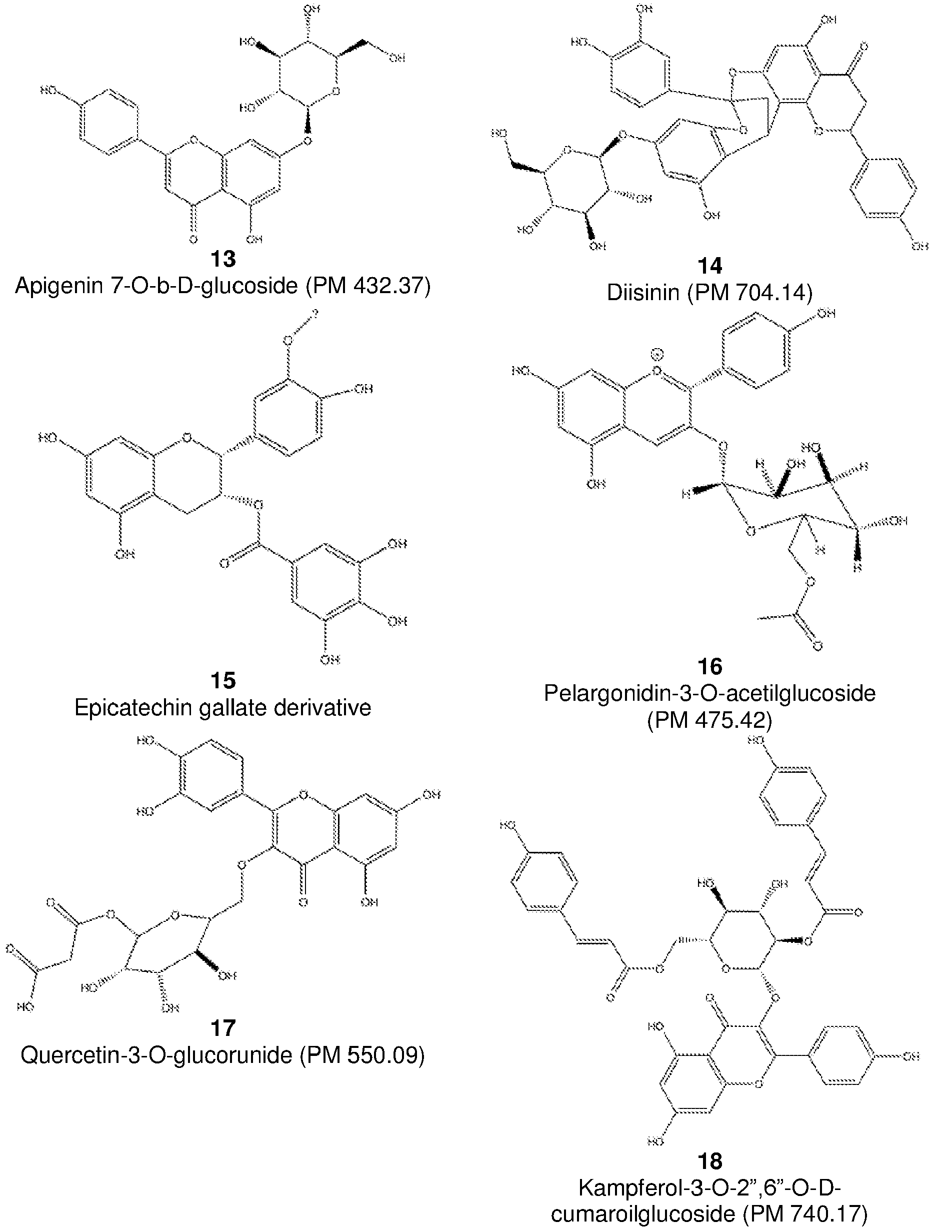

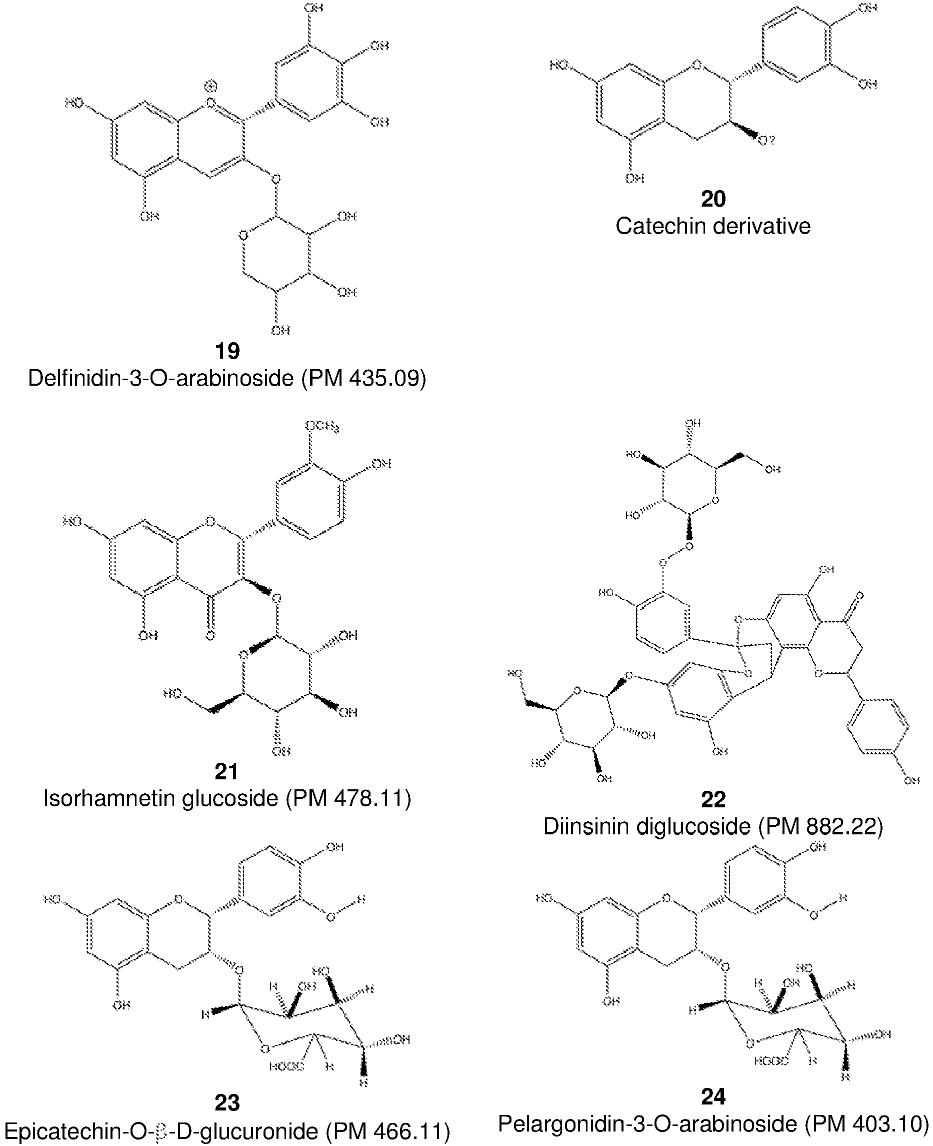

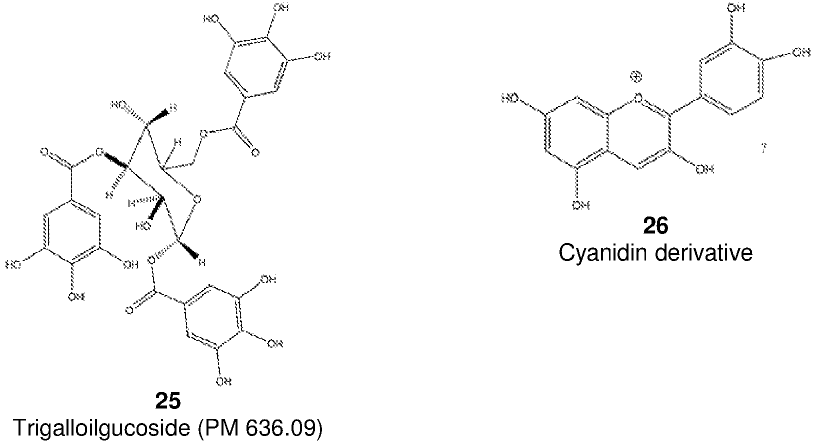

### Others

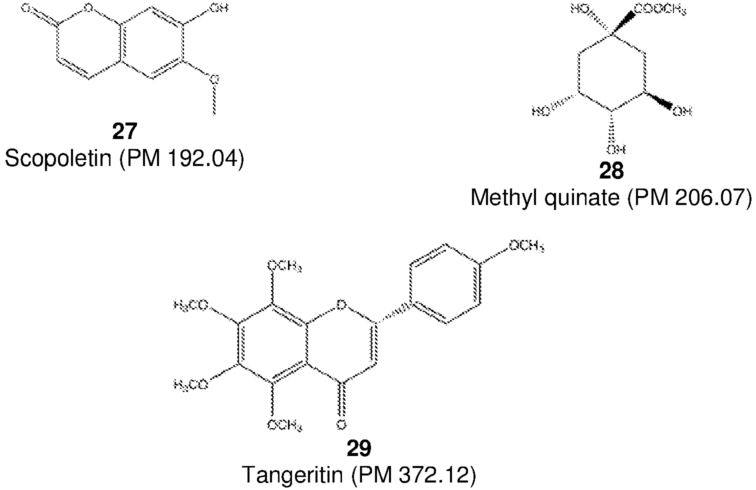

Compounds identified in the extract of *Sambucus nigra* L. according to the invention. In the following paragraph each single category of compounds identified in the extract of the present invention is analyzed, by evaluating the potential biological activity thereof.

### Saponine

In the extract of the invention 2 different saponins were detected: Ginsenoside Rg₁ (**1**) and Thalicoside A (**2**), which constitute about 3% of the identified compounds. These molecules can be traced back to Ginsenosides.

### Acids

This class of compounds existing in the extract is represented by phenolic acids such as the syringic (**3**) and salvianolic acid B (**6**), the quinic acid (**4**) and the betulinic acid (**5**) which is an interesting antibiotic.

### Lignans

In the extract of the invention there are 4 molecules of this class of compounds, which strengthen the immunitary defence and constitute about 9% of the identified compounds: Icariside E4 (**7**), a Diglicoside lignan (**8**), Tianshanoside A (**9**) and a dimer of seicoiolarisiresinol (**10**).

### Polyphenols

The polyphenolic fraction constitutes most part of the compounds included in the extract with a percentage higher than 62%. The relative composition of this fraction is shown in the following Table.

**Polyphenols identified in the extract di Sambucus nigra L.**

| **N. compound** | **Name** | **%** |
|---|---|---|
| **11** | Oleuropein | 2.8 |
| **12** | Quercetin-3-O-(6"-O-malonil)-b-D-glucoside | 5.3 |
| **13** | Apigenin 7-O-β-D-glucoside | 37.9 |
| **14** | Diisinin | 5.8 |
| **15** | Epicatechin gallate derivative | 3.2 |
| **16** | Pelargonidin-3-O-acetilglucoside | 3.3 |
| **17** | Quercetin-3-O-glucorunide | 5.1 |
| **18** | Kampferol-3-O-2",6"-O-D-cumaroilglucoside | 1.6 |
| **19** | Delfinidin-3-O-arabinoside | 1.3 |
| **20** | Catechin derivative | 8.1 |
| **21** | Isorhamnetin glucoside | 6.2 |
| **22** | Diinsinin of glucoside | 7.5 |
| **23** | Epicatechin-O-β-D-glucuronide | 0.9 |
| **24** | Pelargonidin-3-O-arabinoside | 6.1 |
| **25** | Trigalloilgucoside | 4.0 |
| **26** | Cyanidin derivative | 0.9 |

The extract according to the invention is characterized by the presence of some particular compounds such as oleuropein (**11**) which is known as one of the healthy component of olive oil. It is classified as polyphenol, and it is characterized by a glycosidic component and it is considered a "nutraceutic" substance with positive effect against proliferation of tumour cells.

Apigenin glucoside (**13**), constitutes the major component of the mixture of polyphenols in the extract with about 40%. Diisinin (**14**) is a biflavonoid glucoside with marked antiinflammatory activity. Furthermore there is even the corresponding diglucoside (**22**) (about 7.5%) . The presence of these two compounds strengthens significantly the antiinflammatory activity of the extract. Epigallocatechin (**15**) existing in the extract, virtually, could act synergistically with **13** as modulator of the receptors GABA [32].

### Other compounds

In this category two interesting compounds can be found: scopoletin (**27**) and tangeritin (**29**) (existing about 3 and 1%, respectively).

### Examples

The invention consists in the new and original extractive method which allowed a dfferent composition of the extract and then the implementation of a series of cosmetic preparations (thereto the present invention relates too) capable of soothing several skin lesions which cannot be treated wth the decoction obtained according to the procedure shown in the state of art. This is due to the presence of lignans, saponins and antibiotics (see section V) not present in a simple extract obtained as described in the previous patents (see comparison of chromtograms shown in section V). The biomass of ***Sambucus nigra* L.** used by us was collected in Sardinia. The sample is constituted by about 1000 g of aerial portion formed by branches which are not less than two years old deprived of leaves and flowers.

### Extraction with overheated water under pressure

Generally, the extraction from vegetable matrixes of natural active principles contained therein is carried out under mild temperature conditions, so as to avoid the degradation of the thermolable components or the components sensible to the hydrolyitic phenomena.

In literature (PCT/IT2007/000246) the extraction of branches seasoned for 2 years with reflux aqueous solvent is described; based upon the bibliography consolidated in the field of the extraction, such process should be carried out at lower temperature so that the easily degradable substances are preserved. The present invention is based upon the surprising finding that the modification of the extractive process parameters towards more drastic conditions, but in absence of air and under vapour pressure, instead, led to obtain an extract the antioxidant capabilities thereof are unaltered; furthermore the extract obtained by treatment with aqueous solvent under pressure even shows other more marked or innovative properties.

Extraction operating modes: In a 10-lt autoclave 1000 gr of branches, being more than two years old (containing maximum 20-50% of younger branches in vegetative status), were inserted into 8 It deionised water. The water was overheated (120-150 °C) under pressure (2-5 atm), the extraction was carried out for periods of time varying from 1 to 5 hours. After cooling at room temperature (5 - 24 hours), by keeping the autoclave closed, the extract is filtered on filter with porosity so as to prevent the passage of the sediments (1-micron-to-10-micron porosity). The results of the characterization are described in section V.

### Analysis of the extracts

The chromatograms for determining the compounds existing in the extract were obtained with a system by Agilent Technologies (Palo Alto, CA, USA) 1100 series LC/MSD interfaced with mass spectrometer; the tool control and the data processing were performed by Agilent ChemStation HP A.09.01 which under Windows environmente manages the system. In order to be able to determine the existing compounds 300 µL of aqueous extract were used for the LC/MS analysis by using the following system of solvents:
Eluant **A:** acetonitrile HPLC *grade*
Eluant **B:** aqueous solution of trifluoroacetic acid 0.01% (prepared by diluting 50 µL µl of TFA in 500 ml of water MilliQ).

The used column is a Phenomenex Moon C18 (2) 250 x 2.1 mm 5 µm with a precolumn C18 3 µm. Temperature of 33°C. Injection volume 25 µL. The gradient for separating the analytes in mixture is shown in Table:

**Elution gradient**

| **Time (min)** | **Eluant A (%)** | **Eluant B (%)** | **Flow (mL/min)** |
|---|---|---|---|
| 0 | 10 | 90 | 0.5 |
| 20 | 30 | 70 | 0.5 |
| 35 | 43 | 57 | 0.5 |
| 40 | 50 | 50 | 0.5 |
| 60 | 90 | 10 | 0.5 |

**DAD** Dual wavelength reading; λ = 280 nm - 320 nm
**MSD** Optimization conditions ESI; fragmentation voltage CID of 120 V, capillary at 3400 V, "drying gas" N₂ at 350°C at a flow of 10 L/min, with acquisition in negative mode SCAN in the range 150 -800 m/z. The chromatogram is shown in figure 4. Some application examples of these uses are shown hereinafter

### Property of the extract

### Antibacterial and preserving agent

The aqueous extract obtained under most drastic experimental conditions shows a remarkable antibacterial activity, higher than those shown in the International patent PCT/IT2007/000246; in fact in the previous patent it was necessary to add a stabilizing agent (preservative agent) in order to use it in the dermatological formutations whereas this extract does not require it thanks to its chemical composition modified by the conditions of the exctractive process, which make it stable from the microbiological point of view for at least 2 years. Such properties make it suitable to the use of the dermocosmetic formulations in place of the traditional preserving agents with chemical origin (such as parabens, phenoxyethanol, ..).

### Treatment of aphthous stomatitis and inflammation of oral mucosa

The oral mucosa is easily subjected to lesions and wounds caused by factors of different nature which jeopardize the integrity thereof; if such lesions become more serious, as they involve the deepest layers of mucosa, aphthous stomatitis appears, known under the name of "aphtha", the most widespread ulcerative disease. The aqueous extract thereto this patent relates showed to be suitable to the treatment of aphtae of oral cavity, when used as mouth wash with frequency of 2 times a day; the disappearance of aphtae took place after 3 days of topic application.

### Treatment of burning to eves

The burning to eyes is a problem of the organs responsible for viewing which appears with a burning sensation inside and around eyes. Most times the burning is accompanied by other symptoms such as itching, redness, blurred vision, excessive lacrimation, more sensitivity to light, ocular secretions, foreign body sensation.

Under irritation of eyes or, in medical terms, under conjunctivitis the inflammation of conjunctiva, that is the membrane covering externally the eyeball and the inner portion of the eyelids, is meant.

Regardless of the causes of the burning to eyes, the aqueous extract thereto this patent relates showed to be suitable for the treatment of the burning to eyes when it is placed in drops on the eye or nebulized by means of a fine spraying head. The relief is almost immediate and the the burning disappears quickly after one single application.

### Treatment of gastritis

The gastroesophageal reflux, which sometimes is called simply gastric reflux or gastritis, is a problem caused by the temporary reascending of the stomach content in the oesophagus. The gastroesophageal reflux, substantially, is the reascending of the acid content in the oesophagus, the 25-30-cm-long channel which connects the mouth to the stomach; due to its acidity such material irritates the oesophageous mucosa by triggering the typical symptoms of the problem, such as heartburn, acidity and regurgitation.

The aqueous extract thereto this patent relates showed to be suitable for the treatment of the typical symptoms of gastritis if swallowed in quantity of about 50 cc for 3 times a day before meals. After the first day of treatment relief and attenuation of the acid phenomena resulted, which phenomena disappeared completely on the third day of administration by ROS.

### Treatment of irritation of the genital mucosa

The female genital mucosae are particularly delicate and they can be subjected to attacks of outer agents of various nature; the generally present symptoms usually are burning and itching, pain and feeling of tension and, in the most serious cases, it can result that any external agent is capable of irritating the genital region.

The topical treatment of the female genital mucosae with the aqueous extract thereto this patent relates resulted to be successful in the mitigation and disappearance of the symptoms after a couple of days of local application by rinsing.

### Treatment of the symptoms of viral diseases

Herpes labialis is the most widespread pathology in mankind. The responsible agent is Herpes simplex virus of type 1 (HSV-1) and 90% of population carries this virus variant; the infection appears with fever and an erythematosus pharyngitis followed by a vesicular eruption in the oral cavity and on the lip, then afterwards vesicles unify by ulcerating and forming aphtoid patches having grey-yellowish colour.

Chickenpox is a highly contagious infective disease caused by the Chickenpox zoster (Vzv) virus, belonging to the family of Herpes virus. After incubation of 2 or 3 weeks, the disease starts with a skin rash, not high fever and light general symptoms such as discomfort and headache. For 3-4 days, small pink itching papules appear on head, trunk, face and limbs, in subsequent waves. The papules evolve in vesicles, then in pustules and at last in granular crusts, intended to fall. Typically the exanthema is constituted by 250-500 lesions.

Chickenpox, once caught, produces a permanent immunity, but years later it can be followed by herpes zoster. Chickenpox zoster (VZV) virus, in fact, has the capability of remaining latent in the ganglions of the spinnal nerves, invaded during the primary infection, without producing symptoms and, in 10-20% of cases, it can re-activate, by causing the so-called "fire of Saint Anthony" (Herpes zoster). The subject, then, will not have again chickenpox, but a local skin rash characterized by clusters of vesicles which cause a stinging pain along the nerve course, wherein the virus stands. The topical application of the aqueous extract thereto this patent relates on the skin rashes of the pathologies of herpetic viral origin determined in patients a resolution of the symptoms such as burning, pain and skin ailments; an accelerated remission of the skin rash is associated thereto.

### Preparation of products for topical use

The topical preparations, and then even the half-solids preparations for the skin application are vehicles by active principles intended to:
- **Act in surface** (solar creams, repellent, moisturizing, protective agents).
- **Act in the skin appendixes** (anti-acne, disinfectants, products for hair growth).
- **Act in the underneath local tissues** (antifungal, antiinflammatory, antistaminic agents).

### Aqueous spray:

40-60% aqueous extract of *Sambucus nigra* L.
5-10% camomile water
5-10% calendula water
5-10% bluebottle water
5-10% rose water
5-10% orange blossom water
10-30% of Aloe
5-10% of glycerin
0,2-05% of lavender flower oil
0.1-02% of thyme essensial oil

### Gel

30-60% aqueous extract of *Sambucus nigra* L.
5-10% camomile water
5-10% calendula water
5-10% bluebottle water
5-10% rose water
5-10% orange blossom water
5-30% of Aloe
5-20% helichrysum oil
0.2-05% of lavender flower oil
0.1-0.2% of thyme essensial oil
0.1-0.3% rosemary oil

### Mouth wash

50-80% aqueous extract of *Sambucus nigra* L.
5-10% eucaliptus essential oil
5-10% mint essential oil
5-10% lemon essential oil
1-4% thyme essential oil
3-7% calendula water
1-3% rose water
1-3% basil
1-2% sandal

### Purifying face cream

combination and oily skins
20-40% aqueous extract of *Sambucus nigra* L.
7-10% Aloe
3-10% Butyrospermum parkii butter
5-10% Ethylhexyl palmitate
2-5% Cetearyl alcohol
2-5% Caprylic/capric trigliceride
2-5% Glycerin
2-5% Glyceryl stearate citrate
2-5% Sodium pca
2-5% Glyceryl stearate se
2-5% Hydrolyzed wheat protein
2-5% Spiny argan kernel oil
2-5% Olea europaea oil
0.1-0.3% Tetrasodium glutamate diacetate
0.1-0.3% Tocopheryl acetate
0.1-0.3% Sodium dehydroacetate
0.1- 0,20% Allantoin
0.1-0.20% Xanthan gum
0.1% Glyceryl caprylate
0.1% Squalane
0.1% Lecithin
0.1% Tocopheol
0,2-0.7% Ascorbyl palmitate
0,2-0.75% Citric acid
1-2% Benzyl alcohol
0,2-0,8% Dehydroacetic acid
0,2-0.8% Lactic acid
1-2% Lemon essential oil
1-2% Bergamot essential oil
1-4% Propols

### Elasticizing nourishing face cream

20-40% aqueous extract of *Sambucus nigra* L.
7-10% Aloe
3-10% Butyrospermum parkii butter
5-10% Ethylhexyl palmitate
2-5% Cetearyl alcohol
2-5% Caprylic/capric trigliceride
2-5% Glycerin
2-5% Glyceryl stearate citrate
2-5% Sodium pca
2-5% Glyceryl stearate se
2-4% Hydrolyzed wheat protein
2-5% Spiny argan kernel oil
2-5% Olea europaea oil
0.1-0,4% Tetrasodium glutamate diacetate
0.1-0,4% Tocopheryl acetate
0.1-0,4% Sodium dehydroacetate
0.1-0,4% Allantoin
0.1-0,2% Xanthan gum
0.1-0,2% Glyceryl caprylate
0.1-0,2% Squalane
0.1-0,2% Lecithin
0,3-0,8% Tocopheol
0,3-0,8% Ascorbyl palmitate
0,3-0,8% Citric acid
0,5-2% Benzyl alcohol
0,3-0,8% Dehydroacetic acid
0,3-0,8% Lactic acid
3-8% Sweet almond oil
0,3-0,8% Lavender essential oil
0,3-0,8% Pine essential oil

### Liquid soap

Ingredients for about 2 liters (500 gr of base fats):
Olive Oil (50%) 250 gr
Sesame Oil (20%) 100 gr
Wheat Germ oil (20%) 100 gr
Sweet almond oil (10%) 50 gr
Honey 40 ml
Essential oils at choice up to 40 ml (even less or none, it preferred)
Sodium hydroxyde (caustic soda) 62 gr (already deducted by 8%)
Aqueous extract 180 gr

### Personal liquid soap

By using the liquid soap prepared as described, the following formulation was implemented:
- 100 ml of liquid detergent pH 4.5
- 15 drops of lavender flower oil
- 15 g drops of Tea Tree essential oil
- 10 drops of sandal essential oil

### Solid soap

- 1 kg of Olive Oil
- 128 grams of caustic soda (NaOH)
- 300 mL of aqueous extract
- 10 ml of lavender flower oil
- 1 spoonful of potato starch
- 1 spoonful of ground lavender or helicrysum dried flowers

### EXAMPLE OF APPLICATION OF PREPARATIONS

The examples reported hereinafter are the description of application of the preparations by volunteers, who described spontaneously the advantages obtained by the dermocosmetic treatment.

### ABRASIONS AND WOUNDS

Deterge the involved portion and apply the spray, let to act without covering, once dried, apply the gel for three times a day.

Whoever used the product succeeded in solving in 6/8 days

### ACNE AND WRINKLES

Deterge face and neck with the soap and apply the cream about every 8 hours. Several people obtained optimum results, less evident wrinkles and disappered acne

### SORES IN THE MOUTH

Rinse with mouth wash 2 to 3 times a day, after the 3rd rinsing one solves with the disappearance of the pustules

### THROAT PLAQUES

Gargle with mouth wash without food 2 to 3 times per day.

The problem is solved in less than two days.

At least twenty people demonstrate the treatment success

### VARIOUS - TOPICAL DERMATITIS

Apply the gel or cream every 3-4 hours, if the portion to be treated is very delicate, use the spray.

The problem was solved on 5 volunteers.

### DERMATITIS AND DIAPER RASH

Deterge with soap and apply the cream upon changing the diaper, repeat the process upon the following change. Usually two applications are sufficient, upon the first treatment even the spray can be used.

### ECZEMA AND FUNGI

Treat every 8-10 hours with the spray o gel

### INTERNAL FISSURES AND HEMORRHOIDS

Some people used the liquid product by means of the anal syringes, by washing or by using the cream externally and they noted an immediate improvement of pain and a decrease in the volume of haemorrhoids.

### HERPES ZOESTER

Apply the liquid or gel every 4 hours on the involved portions.

### HERPES SIMPLEX

Apply the spray liquid at first and then gel or cream every 6 hours.

### SOLAR ERYTHEMA

Nebulize the liquid at the beginning of the treatment and then continue with gel until the ailment disappears (1-2 hours)

### CHAPPING AND SORES

For cracks caused by allergies to detergents or due to any sore of hands, apply the gel or cream 2 to 3 times a day

### DECUBITUS PLAQUES

As first intervention nebulize the liquid on the sore, then continue the treatment with cream or gel, leaving the portions uncovered, in many cases the product was given (free of charge) to homes for the elderly with bedridden patients, the sores heal up in very short periods, itching and ailments disappear immediately.

### PSORIASIS

Nebulize the liquid as first treatment or when the involved portions result to be very delicate, then apply the gel or cream.

Several people having the problem to the elbows, knees, hands, head, ears and even in intimate parts, obtained advantages, it eliminates itching, it softens the crust, it changes the skin colour and makes it more elastic.

### VARIOUS SKIN RASHES

Nebulize the product or apply the gel every 6/8 hours.

Many people solved problems of skin redness, which cannot be eliminated with cortisone treatment.

### BITES (MOSQUITOS/PAPPATACI/FLEAS)

After the puncture apply the gel, one or two applications are sufficient to reduce swelling and the itching disappears.

The treatment worked in an extraordinary way, surprisingly on the skin of children, with disappearance of itching in few minutes.

### INSECT BITES, WASPS AND BEES

Apply the gel two to three times few minutes after the insect bite, it removes the swelling, itching and pain. It worked both on adults and children.

### SKIN BLEMISHES

It lightens the skin dark blemishes, apply the cream twice a day until absorption. Blemishes which remain after pregnancy.

Some people tested the preparation by following indications and they noted that the blemishes lighten so as to be not recognizable as such.

### EVEN SERIOUS 1-DEGREE BURNS

1-degree burns were treated. Nebulize the liquid firstly, when the skin has dried, apply the gel by covering the whole layer involved by the burn, keep always covered with gel, without ever applying gauzes or bandages, the burnt portion heals up in about 20/26 days.

### CONTACT IRRITATION LIKE MEDUSA

Apply the liquid once or twice, subsequently the gel, it eases the burning sensation immediately and in few hours everything disappears without leaving trace. The treatment was tested on the beach by several volunteers which confirm the great effectiveness.

### CHICKENPOX-RUBELLA

Nebulize the liquid every 3-4 hours or apply the gel, it dries up the pustules without leaving scars typical of pathologies. The treatment was tested both by adults and children with surprising effectiveness.

### REDNESS OF THE EYES

Nebulize at open eyes, it removes inflammation and itching in few minutes.

A person used the liquid to treat a viral conjunctivitis, by making compresses 2 to 3 times a day with sterile gauzes, in one week he/she solved the problem, he/she had a cornea lesion caused by the pathology which was not found any more during the examination by the specialist;

### POST SHAVING SOOTHING

Optimum shoothing and healing agent for post shaving and razor cuts.

Some women who used the soap before depilation, obtained optimum results, softer skin, no burn after depilation, moreover women suffering from folliculitis obtained advantages as anti-inflammatory agent.

### PERIANAL FISTULAS OR SACRO-COCCYGEAL

People suffering from coccygeal and perianal fistulas used the product instead of the usual ointment to favour maturation of fistula and pus discharge, by easing infection pain and by leading to heal the involved portion.

### CHAPPED LIPS

Apply the gel or cream on the lips, it removes chapping and redness. Many people tested the products with quick and very effective results.

### RASHES IN THE HEAD AMONG HAIR

The gel is applied for skin lesions in the head; after some applications they dry up; a woman having lesions not sensible to pharmacological treatments, solved the problem by applying the gel.

### INTIMATE ITCHING AND ANAL ITCHING

One nebulizes with the spray, then the gel is applied and washing with the intimate detergent is performed. The itching disappears shortly after the first application. Volunteers confirm the disappearance of the itching few minutes after the application.

### HAIR REGROWTH

The spray is nebulized and the skin is frictioned in the places wherein there is no hair, after twenty days the regrowth starts, there are several examples on volunteers, especially female volunteers.

The stability tests were carried out in the following way:
A liter of the extract obtained according to the present invention, which in the HPLC analysis shows a standard composition, prepared as usual, kept in dark glass bottle carefully closed with metal screw stopper and kept in fridge at 4°C, was analyzed by HPLC/MS after one month, six months and 12 months. The analysis was performed under the same conditions, by using the same tool and the same operator, no significant variations in the composition appeared, the chromatographic profile remained substantially the same, with variations in the quantity of the components not higher than 3%. From the organolectic point of view the beginning of a slight yellow colour and the appearance of a a slight odour were noted. The microbiological analyses confirmed the absence of bacterical contamination. The chemical composition in polyphenols and phenolic acids practically remained identical, then one can conclude that there is no degradation of the active components of the extract, at least for 12 months under these preservation conditions.

10 extractions were performed 2 days one after the other one with the purpose of determining a possible variability in the composition of the extract in the various batches. The variability resulted to be very limited, the different extracts of Sardinian elder, coming from the same collection station, were analized without determining qualitative differences of composition; some differences in the quantity of the components were detected, but still within 5% of shifting. Even two elder extracts obtained from elder coming from different areas (collected in the Peninsula - Abruzzo, Umbria) were analyzed, in this case the differences in the extract composition are more marked, with some component existing in decidedly different quantities, in each case the main components are the same with variations within the range of 5-10%. This demonstrates the validity of the extraction method according to the present invention.

Based upon the performed experimental tests, the aqueous extract obtained under the experimental conditions of the present invention, more drastic with respect to the previously known ones, shows a remarkable antibacterial activity, well higher than what reported in the International application PCT/IT2007/000246; in fact in the description of the just mentioned application it was necessary to add a stabilizing (preserving) agent in order to use it in the dermatological formulations, whereas the extract of the present invention does not need it thanks to its chemical compositions modified by the conditions of the extractive process, which make it stable from the microbiological point of view for at least 1 year. Such properties make it suitable to the use in the dermocosmetic formulations in place of the traditional preserving agents having chemical origin (such as parabens, phenoxyethano, etc.).

### Documentation on the extract properties

The topical application of the aqueous extract thereto this patent relates on the skin rashes determined in patients a resolution of the symptoms and a complete healing in some cases of even serious burns.

Examples of treatment and properties of the extract according to the present invention in case of hand burned with hot oil, eczema in the scalp and viral conjunctivitis of the eye are shown hereinafter, with reference to the enclosed figures.

### Hand burned with hot oil

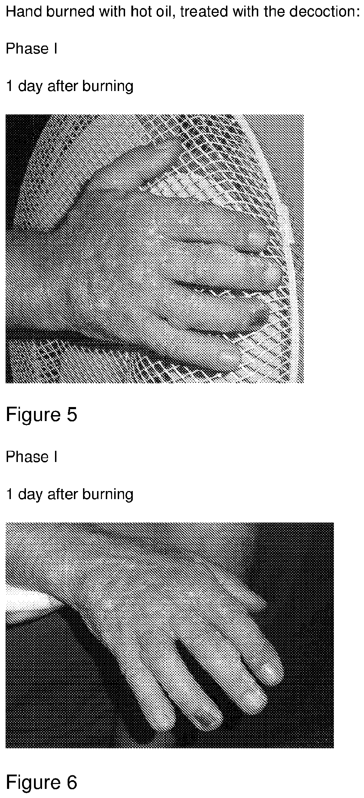

Figure 5 shows a hand burned with hot oil, treated with the extract according to the invention 1 day after burn;
figure 6 the situation of the hand of figure 5 1 day after burn;

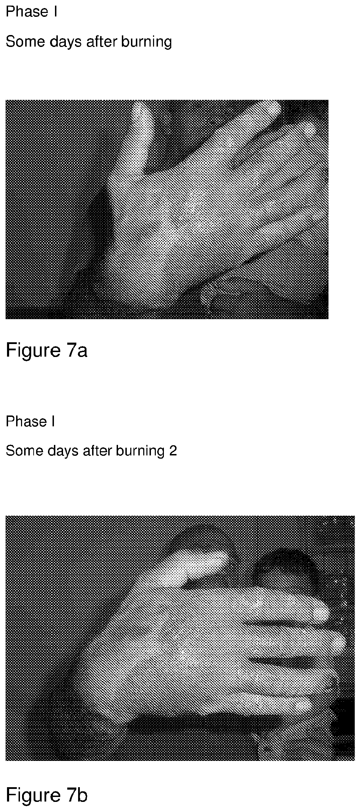

figure 7a and figure 7b the situation of the hand of figure 5 some days after burn;

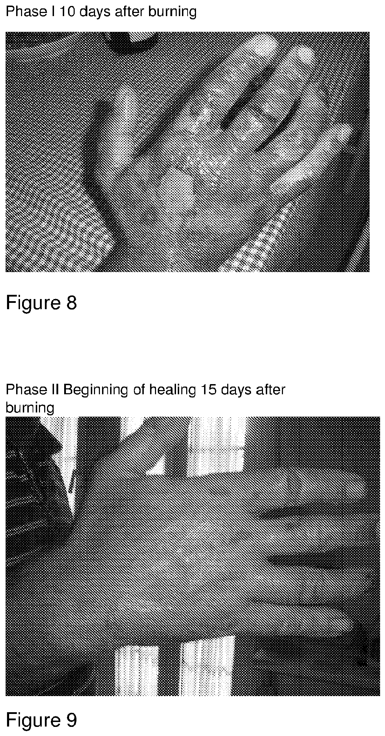

figure 8 the situation of the hand of figure 5 10 days after burn;
Figure 9 the situation of the hand of figure 5 at the beginning of healing 15 days after burn;

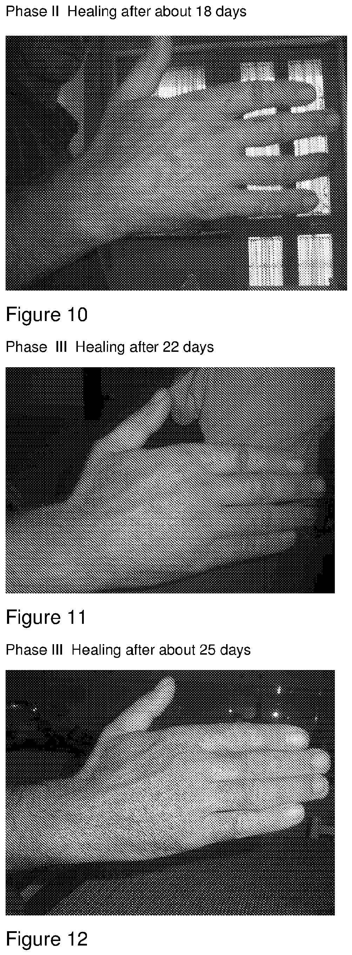

figure 10 the situation of the hand of figure 5 after about 18 days;
figure 11 the situation of the hand of figure 5 showing healing after 22 days;
figure 12 the situation of the hand of figure 5 showing healing after 25 days;

### Eczema in the treated scalp

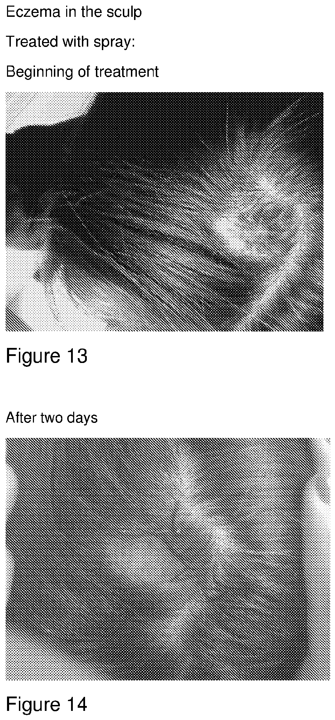

figure 13 shows the scalp with eczema at the beginning of the treatment;
figure 14 shows the scalp after two days of treatment with a spray according to the present invention;

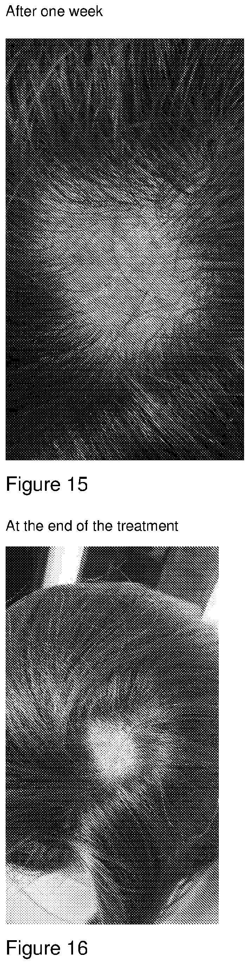

figure 15 shows the scalp after one week of treatment;
figure 16 shows the scalp at the end of treatment;

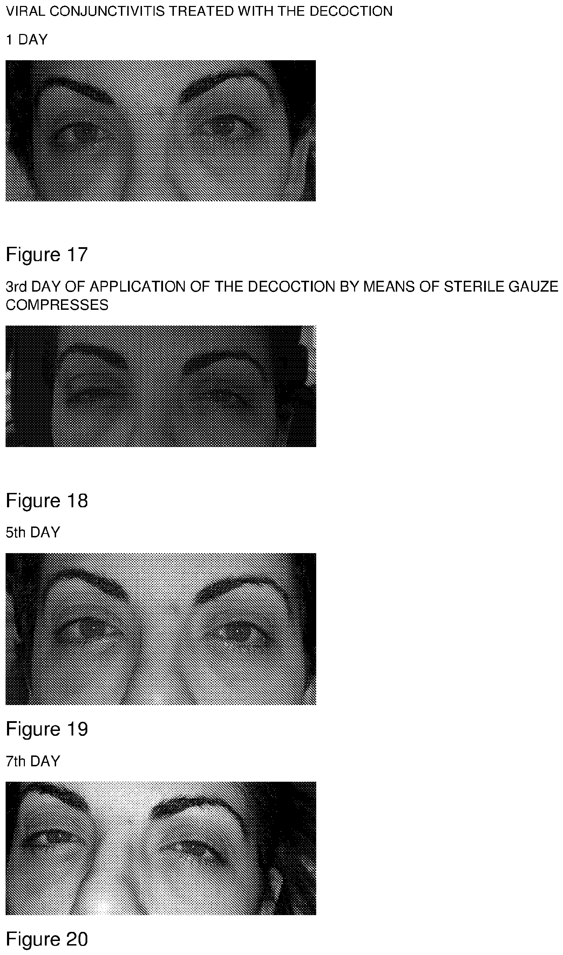

figure 17 shows an eye suffering from viral conjunctivitis treated with decoction according to the present invention 1 day;
figure 18 the eye on the 3rd day of application of a decoction according to the invention by means of sterile gauze compresses;
figure 19 the eye on the 5th day;
figure 20 the eye on the 7th day;

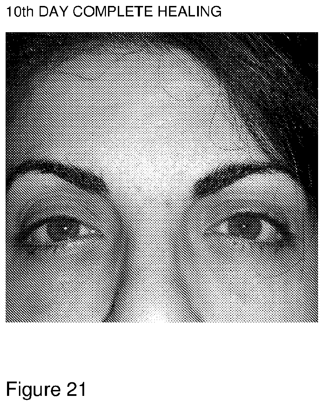

figure 21 the eye on the 10th day after complete healing.
*[1]* Cotton, C., 1996. Ethnobotany: Principles and Applications. Wiley
*[2]* Atzei A.D., 2002. Le piante nella tradizione popolare della Sardegna. CarloDelfino Editore
[3] Bagchi D1, Sen CK, Bagchi M, Atalay M Anti-angiogenic, antioxidant, and anti-carcinogenic properties of a novel anthocyanin-rich berry extract formula*.*
[4] Battelli MG1, Citores L, Buonamici L, Ferreras JM, de Benito FM, Stirpe F, Girbés T. Toxicity and cytotoxicity of nigrin b, a two-chain ribosome-inactivating protein from Sambucus nigra: comparison with ricin. Arch Toxicol. 1997*.*
*[5]* Davidek J. Isolation of chromatographically pure rutin from flowers of elder. Nature 1961
*[6]* Mülleder U1, Murkovic M, Pfannhauser W Urinary excretion of cyanidin glycosides. J Biochem Biophys Methods. 2002 Oct-Nov
*[7]* Toulemonde B and Richard HM. Volatile constituents of dry elder (Sambucus nigra L.) flowers. J Agric Food Chem 1983
*[8]* Scawen MD, Ramshaw JA, Brown RH, Boulter D. The amino-acid sequence of plastocyanin from Sambucus nigra L. (Elder). Eur J Biochem. 1974 May 2
*[9]* Hensel A1, Deters AM, Müller G, Stark T, Wittschier N, Hofmann T. Occurrence of N-phenylpropenoyl-L-amino acid amides in different herbal drugs and their influence on human keratinocytes, on human liver cells and on adhesion of Helicobacter pylori to the human stomach. Planta Med. 2007 Feb;73(2):142-50*. Epub 2007 Feb 13.*
*[10]* Van Damme EJ1, Roy S, Barre A, Rouge P, Van Leuven F, Peumans WJ. The major elderberry (Sambucus nigra) fruit protein is a lectin derived from a truncated type 2 ribosome-inactivating protein. Plant J. 1997 Dec
*[11]* Van Damme EJ1, Roy S, Barre A, Citores L, Mostafapous K, Rouge P, Van Leuven F, Girbés T, Goldstein IJ, Peumans WJ. Elderberry (Sambucus nigra) bark contains two structurally different Neu5Ac(alpha2,6)Gal/GalNAc-binding type 2 ribosome-inactivating proteins. Eur J Biochem. 1997 May
*[12]* Van Damme EJ1, Barre A, Rouge P, Van Leuven F, Peumans WJ Isolation and molecular cloning of a novel type 2 ribosome-inactivating protein with an inactive B chain from elderberry (Sambucus nigra) bark.. J Biol Chem. 1997 Mar
*[13]* Broekaert WF, Nsimba-Lubaki M, Peeters B, Peumans WJ. A lectin from elder (Sambucus nigra L.) bark. Biochem J. 1984 Jul
*[14]* Peumans WJ1, Roy S, Barre A, Rouge P, van Leuven F, van Damme EJ. Elderberry (Sambucus nigra) contains truncated Neu5Ac(alpha-2,6)Gal/GalNAc-binding type 2 ribosome-inactivating proteins. FEBS Lett. 1998 Mar
*[15]* de Benito, F. M., Iglesias, R., Ferreras, J. M., Citores, L., Camafeita, E., Mendez, E., and Girbes, T. Constitutive and inducible type 1 ribosome-inactivating proteins (RIPs) in elderberry (Sambucus nigra L.)*.*
*[16]* Chang WS1, Lee YJ, Lu FJ, Chiang HC. Inhibitory effects of flavonoids on xanthine oxidase. Anticancer Res. 1993 Nov-Dec
*[17] "*Thermal stability, antioxidant activity, and photo-oxidation of natural polyphenols" Irina Volf, loana Ignat*, Mariana Neamtu*, Valentin I. Popa, Chemical Papers 68 (1) 121-129 (2014)
[18] " Thermal Stability of Selected Natural Red Extracts Used as Food Colorants", Jose A. Fernández-López & Jose M. Angosto & Pedro J. Giménez & Gerardo Leon, Plant Foods Hum Nutr (2013) 68:11-17.

## Claims

1. A process for the production of extracts from branches of *Sambucus nigra L.* comprises the following procedures
a. arrangement of two-year-old branches of *Sambucus nigra L.*
b. addition of deionized water and extraction of the material obtained from the procedure a) in an autoclave at pressure between 2 and 5 atm and in the absence of air, for times varying from 1 to 5 hours and at a temperature of 120 - 150°C, using a weight ratio of material obtained from stage a): deionized water from 1:10 to 1:20;
c. cooling to room temperature for a period of 5 - 24 hours and collection of the aqueous extract;
d. filtration on a filter having a porosity such as to prevent the passage of sediment from 1 micron to 10 micron and obtainment of a filtered extract.

2. The process according to claim 1, wherein said two-year-old branches of *Sambucus nigra* L. contain from 20 to 50% by weight of younger branches in vegetative status.

3. The process according to at least one of the preceding claims, wherein said branches in said stage a) are grinded.

4. The process according to at least one of the preceding claims, wherein said pressure is comprised between 3 and 4 atmospheres.

5. An extract obtainable by the process as claimed in at least one of claims 1 to 4.

6. The extract according to claim 5 wherein said extract comprises betulinic acid and lignans.

7. The extract according to claim 5 or 6 for use in the treatment of abrasions and wounds, sores in the mouth, throat plaques, eczema , internal fissures and hemorrhoids, herpes zoster, herpes simplex, decubitus plaques, psoriasis, insect bites, burns, contact irritation, chickenpox-rubella, redness of the eyes, dermatitis, perianal or sacro-coccygeal fistulas, intimate itching and anal itching acne, post shaving soothing, hair therapeutic regrowth, skin blemishes, skin rashes, chapped lips, solar erythema chapping and sores.

8. A composition containing the extract as claimed in claims 5 or 6 and one or more tolerable additives for use in the treatment of abrasions and wounds, sores in the mouth, throat plaques, eczema, internal fissures and hemorrhoids, herpes zoster, herpes simplex, decubitus plaques, psoriasis, insect bites, burns, contact irritation, chickenpox-rubella, redness of the eyes, dermatitis, perianal or sacro-coccygeal fistulas, , intimate itching and anal itching, acne, post shaving soothing, therapeutic hair regrowth, skin blemishes, skin rashes, chapped lips, solar erhythema, chapping and sores.

9. The use of the extract according to claim 5 or 6 for the cosmetic treatment of wrinkles

10. A compositions containing the extract according to claim 5 or 6 and one or more tolerable additives.

## Patentansprüche

1. Verfahren zur Herstellung von Extrakten aus Zweigen von *Sambucus nigra L.* umfassend die folgenden Prozeduren
a. das Anordnen von zwei Jahre alten Zweigen von *Sambucus nigra L.*
b. das Hinzufügen von deionisiertem Wasser und die Extraktion des bei der Prozedur a) erhaltenen Materials in einem Autoklaven bei einem Druck zwischen 2 und 5 Atmosphären und in der Abwesenheit von Luft, für Zeiten zwischen 1 und 5 Stunden und bei einer Temperatur von 120 bis 150 °C, unter Verwendung eines Gewichtsverhältnisses des aus Stufe a) erhaltenen Materials: deionisiertes Wasser von 1:10 bis 1:20;
c. das Abkühlen auf Raumtemperatur für einen Zeitraum von 5 bis 24 Stunden und das Einsammeln des wässrigen Extrakts;
d. die Filtration auf einem Filter mit einer Porosität, die die Passage von Sediment zwischen 1 Mikron bis 10 Mikron verhindert, und das Erhalten eines gefilterten Extrakts.

2. Verfahren gemäß Anspruch 1, wobei die zwei Jahre alten Zweige von *Sambucus nigra L.* zwischen 20 bis 50 Gewichtsprozent an jüngeren Zweigen in vegetativem Status enthalten.

3. Verfahren gemäß einem der vorhergehenden Ansprüche, wobei die Zweige in Stufe a) zerkleinert sind.

4. Verfahren gemäß einem der vorhergehenden Ansprüche, wobei der Druck zwischen 3 und 4 Atmosphären liegt.

5. Extrakt, der durch das Verfahren gemäß wenigstens einem der Ansprüche 1 bis 4 erhalten wird.

6. Extrakt gemäß Anspruch 5, wobei der Extrakt Betulinsäure und Lignane umfasst.

7. Extrakt gemäß Anspruch 5 oder 6 zur Verwendung bei der Behandlung von Schürfwunden und Wunden, wunden Stellen im Mund, Rachenbelägen, Ekzemen, inneren Fissuren und Hämorriden, Herpes-Zoster, Herpes-Simplex, Dekupitusbelägen, Psoriasis, Insektenstiche, Verbrennungen, Kontaktreizungen, Windpocken-Röteln, Rötungen der Augen, Dermatitis, perianale oder sacrococcygeale Fisteln, intimen Juckreiz und analer juckender Akne, Beruhigung nach der Rasur, zum therapeutischen Nachwachsen von Haaren, Hautunreinheiten, Hautausschlägen, rissigen Lippen, Sonnenbrand, Rissen und Wunden.

8. Komposition enthaltend den Extrakt gemäß Anspruch 5 oder 6 und ein oder mehrere tolerierbarer Zusätze zur Verwendung bei der Behandlung von Schürfwunden und Wunden, wunden Stellen im Mund, Rachenbelägen, Ekzemen, inneren Fissuren und Hämorriden, Herpes-Zoster, Herpes-Simplex, Dekupitusbelägen, Psoriasis, Insektenstiche, Verbrennungen, Kontaktreizungen, Windpocken-Röteln, Rötungen der Augen, Dermatitis, perianale oder sacrococcygeale Fisteln, intimen Juckreiz und Analjucken, Akne, Beruhigung nach der Rasur, zum therapeutischen Nachwachsen von Haaren, Hautunreinheiten, Hautausschlägen, rissigen Lippen, Sonnenbrand, Rissen und Wunden.

9. Verfahren zur Verwendung des Extrakts gemäß Anspruch 5 oder 6 zur kosmetischen Behandlung von Falten.

10. Komposition enthaltend den Extrakt gemäß den Ansprüchen 5 oder 6 und ein oder mehrere tolerierbare Zusätze.

## Revendications

1. Procédé pour la production d'extraits de branches de *Sambucus nigra L.,* comprenant les procédures suivantes :
a. obtention de branches de *Sambucus nigra L.* âgées de deux ans,
b. addition d'eau désionisée et extraction du matériau obtenu à partir de la procédure a) dans un autoclave sous une pression comprise entre 2 et 5 atm et en l'absence d'air, pendant des temps variant de 1 à 5 heures et à une température de 120 à 150 °C, utilisant un rapport en poids du matériau obtenu dans l'étape a) à l'eau désionisée de 1/10 à 1/20 ;
c. retour à la température ambiante sur une période de 5 à 24 heures et collecte de l'extrait aqueux ;
d. filtration sur un filtre ayant une porosité empêchant le passage de sédiments de 1 micromètre à 10 micromètres et obtention d'un extrait filtré.

2. Procédé selon la revendication 1, dans lequel lesdites branches de *Sambucus nigra L.* âgées de deux ans contiennent de 20 à 50 % en poids de branches plus jeunes à l'état végétatif.

3. Procédé selon au moins l'une des revendications précédentes, dans lequel lesdites branches dans ladite étape a) sont broyées.

4. Procédé selon au moins l'une des revendications précédentes, dans lequel ladite pression est comprise entre 3 et 4 atmosphères.

5. Extrait pouvant être obtenu par le procédé selon au moins l'une des revendications 1 à 4.

6. Extrait selon la revendication 5, dans lequel ledit extrait comprend de l'acide bétulinique et des lignanes.

7. Extrait selon la revendication 5 ou 6 destiné à être utilisé dans le traitement d'abrasions et de blessures, de plaies dans la bouche, de plaques de la gorge, d'un eczéma, de fissures internes et d'hémorroïdes, d'un herpès zoster, d'un herpès simplex, de plaques de décubitus, d'un psoriasis, de piqûres d'insectes, de brûlures, d'une irritation de contact, de la varicelle-rubéole, d'une rougeur oculaire, d'une dermatite, de fistules périanales ou sacro-coccygiennes, de démangeaisons intimes et de démangeaisons anales, de l'acné, pour un apaisement après le rasage, pour la repousse thérapeutique des cheveux, des imperfections de la peau, des éruptions cutanées, des lèvres gercées, d'un érythème solaire, d'une gerçure et de plaies.

8. Composition contenant l'extrait selon la revendication 5 ou 6 et un ou plusieurs additifs tolérables destinés à être utilisés dans le traitement d'abrasions et de blessures, de plaies dans la bouche, de plaques de la gorge, d'un eczéma, de fissures internes et d'hémorroïdes, d'un herpès zoster, d'un herpès simplex, de plaques de décubitus, d'un psoriasis, de piqûres d'insectes, de brûlures, d'une irritation de contact, de la varicelle-rubéole, d'une rougeur oculaire, d'une dermatite, de fistules périanales ou sacro-coccygiennes, de démangeaisons intimes et de démangeaisons anales, de l'acné, pour un apaisement après le rasage, pour la repousse thérapeutique des cheveux, des imperfections de la peau, des éruptions cutanées, des lèvres gercées, d'un érythème solaire, d'une gerçure et de plaies.

9. Utilisation de l'extrait selon la revendication 5 ou 6 pour le traitement cosmétique de rides.

10. Composition contenant l'extrait selon la revendication 5 ou 6 et un ou plusieurs additifs tolérables.
